# EUROPÄISCHE PATENTSCHRIFT

(11) **EP 0 934 925 B1**
(45) Veröffentlichungstag und Bekanntmachung des Hinweises auf die Patenterteilung: **25.09.2002**
(21) Anmeldenummer: 98122408.2
(22) Anmeldetag: 26.11.1998
(51) Int. Cl.: C07C 253/30, C07C 213/02, C07C 255/13, C07C 217/08

(54) **Verfahren zur Herstellung von Beta-Alkoxy-nitrilen**
Process for the preparation of beta-alkoxy nitriles
Procédé pour préparer des nitriles beta-alkoxy

(30) Priorität: 30.01.1998 DE 19803515
(43) Veröffentlichungstag der Anmeldung: 11.08.1999
(73) Patentinhaber: BASF AKTIENGESELLSCHAFT, 67056 Ludwigshafen (DE)
(72) Erfinder: Friedrich, Wolfgang, 67346 Speyer (DE); Kneuper, Heinz-Josef Dr., 68163 Mannheim (DE); Eller, Karsten Dr., 67061 Ludwigshafen (DE); Henne, Andreas Dr., 67433 Neustadt (DE); Lebkücher, Rolf Dr., 68165 Mannheim (DE)

(56) Entgegenhaltungen:
- DE-A- 2 136 884
- W. P. UTERMOHLEN: "Preparation of gamma-Alkoxy-n-propylamines" JOURNAL OF THE AMERICAN CHEMICAL SOCIETY., Bd. 67, Nr. 9, 13. September 1945, Seiten 1505-1506, XP002103505 DC US
- DATABASE WPI Section Ch, Week 9310 Derwent Publications Ltd., London, GB; Class A11, AN 93-080428 XP002103506 & JP 05 025201 A (NIPPON KASEI KK) , 2. Februar 1993

## Beschreibung

Die Erfindung betrifft ein Verfahren zur Herstellung von β-Alkoxy-nitrilen durch Umsetzung von niedermolekularen α,β-ungesättigten Nitrilen, z. B. mit bis zu 40 C-Atomen, mit ein-, zweioder dreiwertigen Alkoholen, z. B. mit jeweils einer Molmasse von bis zu 2,5 • 10³ g/Mol, in Gegenwart basischer Katalysatoren bei Temperaturen von -20 bis +200 °C.

Die 1,4-Addition von ein- oder mehrwertigen Alkoholen an α,β-ungesättigte Nitrile ist eine bekannte Reaktion und wird aufgrund des Reaktionsmechanismus' in J. March, Advanced Organic Chemistry, 3^{rd} Ed., Seite 665, J. Wiley & Sons, 1985, als eine 'Michael-type-addition' bezeichnet.

Wie zum Beispiel in H. A. Bruson, Organic Reactions, Vol. 5, Chapt. 2, Seite 89, R. Adams (Ed.), J. Wiley, 1949, ausgeführt, benötigt diese Additionsreaktion gewöhnlicherweise einen basischen Katalysator, um befriedigende Reaktionsgeschwindigkeiten zu erzielen.

In vielen Fällen werden die Reaktionsmischungen, die das 1,4-Additionsprodukt enthalten, ohne Reinigungsmaßnahmen direkt in einem zweiten Verfahrensschritt durch eine anschließende katalytische Hydrierung in γ-Alkoxy-amine überführt. Eine Übersicht über die bekannten Verfahren findet sich beispielsweise in Houben-Weyl, Methoden der organischen Chemie, Band 11/1, Seite 341 ff, 4. Auflage (1957).

Da die 1,4-Addition von Alkoholen an α,β-ungesättigte Nitrile zu β-Alkoxy-nitrilen reversibel ist, gilt es zu vermeiden, daß in der evtl. nachgeschalteten Hydrierstufe in Gegenwart des basischen Katalysators eine Rückspaltung des β-Alkoxy-nitrils stattfindet (vergl. auch: H. A. Bruson, Organic Reactions, Vol. 5, Seite 90, 3. Absatz, Zeilen 8-11). Eine Abtrennung der in geringen Mengen eingesetzten basischen Katalysatoren vor der Hydrierstufe ist unwirtschaftlich und deshalb müssen sie mit einer Säure neutralisiert werden. In der evtl. nachgeschalteten katalytischen Hydrierung des β-Alkoxy-nitrils darf es nicht zu einer Schädigung des Hydrierkatalysators durch den 1,4-Additions-Katalysator bzw. seine neutralisierte Form kommen.

Typische Katalysatoren für die 1,4-Addition von Alkoholen an α,β-ungesättigte Nitrile sind, wie ebenfalls in H. A. Bruson, Organic Reactions, Vol. 5, Seite 81 und 89 ausgeführt, beispielsweise die Metalle Natrium und Kalium oder deren Oxide, Hydroxide, Hydride, Cyanide und Amide. Die Katalysatoren werden üblicherweise in Mengen von 0,5 bis 5 Gew.-%, bezogen auf den Alkohol, eingesetzt.

In W.P. Utermohlen, J. Am. Chem. Soc. 67, 1505-6, wird Natriummethanolat als basischer Katalysator verwendet.

Der Einsatz von Alkalimetallen bringt erhebliche Probleme mit der Handhabung dieser reaktiven Katalysatoren mit sich. Weiterhin sind auch Alkalimetall-hydride, -amide und -alkoholate sehr feuchtigkeitsempfindlich und nur unter aufwendigen Maßnahmen technisch handhabbar. Eine Kontrolle der chemischen Zusammensetzung dieser Katalysatoren vor deren Einsatz zur Ermittlung ihrer Aktivität ist unerläßlich.

Es hat daher nicht an Versuchen gefehlt, Katalysatoren zu finden, die im technischen Maßstab einfach handhabbar sind und zugleich eine genügend hohe Aktivität aufweisen, um die 1,4-Additionsreaktion mit möglichst guten Raum-Zeit-Ausbeuten zu ermöglichen.

Die DE-A-20 61 804 beschreibt, daß bei der 1,4-Addition von β-thio- oder β-sulfoxid-substituierten Ethanolen an α,β-ungesättigte Nitrile als basische Katalysatoren u.a. organische sekundäre oder tertiäre Amine, wie zum Beispiel Piperidin oder Triethylamin, eingesetzt werden können. Sekundäre Amine sind jedoch nur bedingt als Katalysatoren geeignet, da sie selbst mit α,β-ungesättigten Nitrilen reagieren.

In der DE-A-35 22 906 wird erwähnt, daß sowohl für die Herstellung von 2,2'-Dicyanodiethylether (NC-(CH₂)₂-O-(CH₂)₂-CN) aus Acrylnitril und Wasser als auch für die Synthese von β-Alkoxy-nitrilen aus 2,2'-Dicyanodiethylether und einem Alkohol als basische Katalysatoren u.a. tertiäre Amine, wie zum Beispiel Triethylamin oder Pyridin, verwendet werden können.

Für die 1,4-Addition von Formaldehydcyanhydrin an Acrylnitril unter Bildung von β-(Cyanomethoxy)-propionitril wurde in der US 2,333,782 Tributylamin als Katalysator offenbart.

Als basische Katalysatoren für die 1,4-Addition von Alkoholen an α,β-ungesättigte Nitrile sind häufig quartäre Tetraalkylammoniumhydroxide bzw. Lösungen hiervon, wie zum Beispiel Benzyltrimethylammoniumhydroxid (= Triton® B), beispielsweise beschrieben in US 3,493,598 und W. P. Utermohlen, J. Am. Chem. Soc. 67, 1505-6 (1945), oder Tetrakis-(2-hydroxyethyl)-ammoniumhydroxid, beispielsweise beschrieben in DE-A-21 21 325 und DE-A-22 17 494, eingesetzt worden.

Wie allgemein bekannt ist, sind Tetraalkylammoniumhydroxide thermisch nicht stabil und zersetzen sich zu einem Trialkylamin, Alken und Wasser (Hofmann-Eliminierung; siehe z. B.: R.T. Morrison und R.N. Boyd, Organic Chemistry, 6^{th} Ed., 1992, Seite 854 unten bis Seite 855 oben).

Auch Tetraalkylammoniumhydroxide mit 1 bis 4 β-Hydroxy-Substituenten sind thermisch instabil und zersetzen sich durch intraund/oder intermolekulare Reaktionen (siehe z. B.: A. R. Doumaux et al., J. Org. Chem. 38, 3630-2 (1973) und A. C. Cope et al. in 'Organic Reactions', Vol. 11, Chapter 5, Wiley, New York, 1960).

Daher sind diese Katalysatoren bzw. deren Lösungen nur bedingt lagerfähig und es ist auch hier eine Kontrolle ihrer chemischen Zusammensetzung vor deren Einsatz zur Ermittlung ihrer Aktivität erforderlich.

Aufgrund der thermischen Labilität von Tetraalkylammoniumhydroxiden sind bei ihrem Einsatz als Katalysatoren für die 1,4-Addition von Alkoholen an α,β-ungesättigte Nitrile bei den üblichen Reaktionstemperaturen von 35 bis 140 °C (H. A. Bruson, Organic Reactions, Vol. 5, Chapt. 2, Seite 89, 90 und 93) die Ausbeuten an den 1,4-Additionsprodukten oft verbesserungsbedürftig. Ein gewichtiger Nachteil ist weiterhin die Tatsache, daß ein thermisch teilweise zersetzter Katalysator bzw. dessen Lösung ein verspätetes Anspringen der 1,4-Additionsreaktion bewirkt. Dies kann zur Folge haben, daß sich im Reaktionsgefäß, in dem die Additionsreaktion durch Zugabe des α,β-ungesättigten Nitrils, z. B. Acrylnitril, zum Alkohol durchgeführt wird, eine sicherheitstechnisch gefährlich hohe Nitril-Konzentration einstellt, die im Extremfall zu einer Polymerisation des α,β-ungesättigten Nitrils unter starker Wärmeentwicklung führen kann.

Weitere Nachteile der quartären Ammoniumhydroxide sind ihre Nichteinsetzbarkeit bei der 1,4-Addition von mehrwertigen Alkoholen an α,β-ungesättigte Nitrile (siehe DE-A-22 17 494), die Tatsache, daß die 1,4-Additionsprodukte oftmals eine unerwünschte Verfärbung aufweisen, und die Notwendigkeit, sie nach erfolgter 1,4-Additionsreaktion durch Säurezugabe zu neutralisieren und das entstehende Salz abzutrennen, wenn die β-Alkoxy-nitrile direkt einer katalytischen Hydrierung zur Gewinnung von γ-Alkoxy-aminen unterworfen werden sollen (siehe DE-A-21 36 884).

Aufgabe der vorliegenden Erfindung war es deshalb, ein verbessertes Verfahren zur 1,4-Addition von ein-, zwei- oder dreiwertigen Alkoholen an α,β-ungesättigte Nitrile zu finden, das die oben beschriebenen Nachteile nicht aufweist und das es auch ermöglicht, die erhaltene Reaktionsmischung, die die 1,4-Additionsprodukte enthält, direkt in einem zweiten Verfahrensschritt durch eine Hydrierung in Gegenwart eines Hydrierkatalysators in γ-Alkoxy-amine zu überführen, ohne daß eine vorherige Abtrennung oder Neutralisation des Katalysators für die 1,4-Addition notwendig ist.

Demgemäß wurde ein Verfahren zur Herstellung von β-Alkoxy-nitrilen durch Umsetzung von α,β-ungesättigten Nitrilen mit beispielsweise 3 bis 40 C-Atomen mit ein-, zwei- oder dreiwertigen Alkoholen mit beispielsweise jeweils einer Molmasse von bis zu 2,5 • 10³ g/Mol in Gegenwart basischer Katalysatoren bei Temperaturen von -20 bis +200 °C gefunden, das dadurch gekennzeichnet ist, daß man als Katalysator ein Diazabicycloalken der Formel I 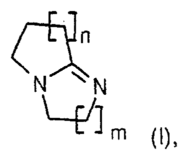 in der 1 bis 4 H-Atome unabhängig voneinander durch die Reste R¹ bis R⁴, wobei
R¹, R², R³, R⁴ C₁₋₂₀-Alkyl, C₆₋₂₀-Aryl oder C₇₋₂₀-Arylalkyl bedeuten,
substituiert sein können sowie n und m ganze Zahlen von 1 bis 6 bedeuten, verwendet.

Die Reste R¹, R², R³ und R⁴ haben unabhängig voneinander folgende Bedeutung:
- C₁₋₂₀-Alkyl, wie Methyl, Ethyl, n-Propyl, n-Butyl, iso-Butyl, tert.-Butyl, n-Pentyl, iso-Pentyl, Cyclopentyl, Cyclopentylmethyl, n-Hexyl, iso-Hexyl, Cyclohexyl, n-Heptyl, iso-Heptyl, Cyclohexylmethyl, n-Octyl, iso-Octyl, n-Nonyl, n-Decyl, n-Undecyl, n-Dodecyl, n-Tridecyl, n-Tetradecyl, n-Pentadecyl,
   bevorzugt C₁- bis C₈-Alkyl, wie Methyl, Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Dimethylpropyl, n-Hexyl, iso-Hexyl, sec.-Hexyl, Cyclohexyl, Cyclopentylmethyl, n-Heptyl, iso-Heptyl, Cyclohexylmethyl, n-Octyl, iso-Octyl,
   besonders bevorzugt C₁- bis C₄-Alkyl, wie Methyl, Ethyl, n-Propyl, iso-Propyl, n-Butyl, iso-Butyl, sec.-Butyl und tert.-Butyl,
- C₆₋₂₀-Aryl, wie Phenyl, 1-Naphthyl, 2-Naphthyl, 1-Anthryl, 2-Anthryl, 9-Anthryl bevorzugt Phenyl, 1-Naphthyl, 2-Naphthyl, besonders bevorzugt Phenyl,
- C₇₋₂₀-Arylalkyl, bevorzugt C₇₋₁₂-Phenylalkyl, wie Benzyl, 1-Phenethyl, 2-Phenethyl, 1-Phenylpropyl, 2-Phenylpropyl, 3-Phenylpropyl, 1-Phenylbutyl, 2-Phenylbutyl, 3-Phenylbutyl und 4-Phenylbutyl, besonders bevorzugt Benzyl.

Die genannten Reste können unter den Reaktionsbedingungen inerte Substituenten tragen, wie eine oder mehrere Alkylreste, z. B. Methyl, Ethyl, n-Propyl, iso-Propyl, n-Butyl, iso-Butyl, sec.-Butyl, tert.-Butyl.

Die Indices n und m in der Formel I bedeuten unabhängig voneinander jeweils ganze Zahlen von 1 bis 6. Bevorzugt bedeutet n eine ganze Zahl von 1 bis 3 und m eine ganze Zahl von 1 bis 3. Besonders bevorzugt bedeutet n 1, 2 oder 3 und m ist gleich 2.

Als Beispiele für Katalysatoren der Formel I seien genannt:
1,5-Diazabicyclo[4.3.0]nonen-5 (DBN), 1,8-Diazabicyclo[5.4.0]undecen-7 (DBU), 1,6-Diazabicyclo[5.5.0]dodecen-6,
1,7-Diazabicyclo[6.5.0]tridecen-7, 1,8-Diazabicyclo[7.4.0]tridecen-8, 1,8-Diazabicyclo[7.5.0]tetradecen-8,
1,5-Diazabicyclo[4.4.0]decen-5 (DBD), 1,8-Diazabicyclo[5.3.0]decen-7, 1,10-Diazabicyclo[7.3.0]dodecen-9,
1,10-Diazabicyclo[7.4.0]tridecen-9, 2-Methyl-1,5-diazabicyclo[4.3.0]nonen-5, 3-Methyl-1,5-diazabicyclo[4.3.0]nonen-5,
7-Methyl-1,5-diazabicyclo[4.3.0]nonen-5, 7-Benzyl-1,5-diazabicyclo[4.3.0]nonen-5, 11-Methyl-1,8-diazabicyclo[5.4.0]undecen-7,
10-Methyl-1,8-diazabicyclo[5.4.0]undecen-7, 6-Methyl-1,8-diazabicyclo[5.4.0]undecen-7, 6-Benzyl-1,8-Diazabicyclo[5.4.0]undecen-7,
2-Methyl-1,5-diazabicyclo[4.4.0]decen-5, 3-Methyl-1,5-diazabicyclo[4.4.0]decen-5, 7-Methyl-1,5-diazabicyclo[4.4.0]decen-5,
7-Benzyl-1,5-diazabicyclo[4.4.0]decen-5.

Bevorzugt sind DBN, DBD und DBU, besonders bevorzugt sind DBU und DBN, da diese Verbindungen leicht zugänglich sind.

Auch Mischungen von Verbindungen der Formel I, z. B. eine Mischung bestehend aus DBU und DBN, sind als Katalysatoren einsetzbar.

Die erfindungsgemäßen Katalysatoren der Formel I sind im Gegensatz zu den bisher als Katalysator verwendeten quartären Ammoniumverbindungen auch gut für die vollständige Umsetzung aller Hydroxylgruppen von zwei- bzw. dreiwertigen Alkoholen durch 1,4-Addition an α,β-ungesättigte Nitrile einsetzbar.

Ein weiterer Vorteil des erfindungsgemäßen Verfahrens ist, daß die 1,4-Additionsprodukte, also die β-Alkoxy-nitrile, wesentlich geringere Verfärbungen aufweisen als bei der Verwendung der quartären Ammoniumverbindungen des Stands der Technik als Katalysatoren.

Diazabicycloalkene der Formel I können nach verschiedenen Methoden hergestellt werden. Bekannt ist zum Beispiel die Addition von Acrylnitril an Lactame unter Bildung von Cyanoethyl-lactamen, die anschließend zu Aminopropyl-lactamen hydriert und zuletzt unter sauer katalysierter Wasserabspaltung zu den Diazabicycloalkenen zyklisiert werden (H. Oediger et al., Synthesis, S. 591-8 (1972); H. Oediger et al., Chem. Ber. 99, S. 2012-16 (1966).; L. Xing-Quan, J. Nat. Gas Chem. 4, S. 119-27(1995)).

Die ältere deutsche Patentanmeldung Nr. 19752935.6 beschreibt ein Verfahren zur Herstellung von Diazabicycloalkenen durch Umsetzung von Lactonen mit Diaminen unter Wasserabspaltung.

Diazabicycloalkene der Formel I kommen als starke Basen, die aufgrund ihrer geringen Nukleophilie gegenüber gewöhnlichen tert. Aminen wie z. B. Triethylamin oder N,N-Dimethylanilin eine besondere Stellung einnehmen, in einer Reihe organischer Reaktionen zur Anwendung, beispielsweise in Halogenwasserstoff-Eliminierungsreaktionen (siehe obengenannte Lit.).

Die hervorragende Aktivität von Diazabicycloalkenen der Formel I als Katalysatoren für die 1,4-Addition von Alkoholen an α,β-ungesättigte Nitrile ist insofern überraschend, als aus der JP-A-5-25201/93 (Beispiel 1) bekannt war, daß für die Reaktion von bereits zu 85 % cyanethyliertem Pullulan mit Acrylnitril (ACN) ein extrem hoher Überschuß von ca. 30 Mol ACN pro Mol Hydroxylgruppe, eine große Menge von 6 Gew.-% des Katalysators 1,8-Diazabicyclo[5.4.0]undecen-7 (DBU) sowie eine lange Reaktionszeit von zehn Tagen notwendig waren.

Pullulan ist ein aus D-Maltotriose-Einheiten aufgebautes Polymer mit einer Molmasse im Bereich von 5 • 10⁴ bis 2 • 10⁶ g/Mol (Lit.: A. Jeanes in 'Extracellular Microbial Polysaccharides' (P.A. Sandford und A. Laskin, Ed.), Seite 288, 289 und 292, Am. Chem. Soc., Washington, DC (1977)).

Das erfindungsgemäße Verfahren läßt sich wie folgt durchführen:

Bei einer diskontinuierlichen Verfahrensweise wird der Alkohol zusammen mit dem Katalysator der Formel I, gegebenenfalls in einen inerten Lösungsmittel gelöst, in einem Reaktionsgefäß unter Normaldruck oder erhöhtem Druck vorgelegt und das α,β-ungesättigte Nitril zudosiert. Als Reaktionsgefäße kommen beispielsweise Rührreaktoren oder Rührbehälterkaskaden in Betracht.

Bei einem kontinuierlichen Verfahren werden beispielsweise Rührbehälter-, Schlaufen- oder Rohrreaktoren eingesetzt, oder eine Kombination dieser Reaktoren, gegebenenfalls bei jeweils unterschiedlichen Temperaturen, in die die beiden Reaktanden und der Katalysator der Formel I hineinbefördert werden.

Bevorzugt ist unter sicherheitstechnischen Gesichtspunkten eine kontinuierliche Verfahrensweise.

Durch die hohe Aktivität der Katalysatoren der Formel I setzt die Reaktion unter Wärmeentwicklung und Bildung der β-Alkoxynitrile ohne Verzögerung ein und die Konzentration an α,β-ungesättigtem Nitril im Reaktionsgefäß wird somit sehr niedrig gehalten, da es sofort mit dem Alkohol abreagiert. Dies wirkt sich zum einen vorteilhaft auf die Selektivität der 1,4-Additionsreaktion aus und zum anderen werden die oben beschriebenen Sicherheitsrisiken durch Polymerisationsreaktionen von unumgesetztem α,β-ungesättigten Nitril vermieden.

Der Katalysator kann vorteilhaft in einer Menge von 0,05 bis 5 Gew.-%, bezogen auf den Alkohol, eingesetzt werden. Bei geringeren Mengen werden längere Reaktionszeiten oder höhere Reaktionstemperaturen benötigt, größere Mengen sind aus wirtschaftlichen Gründen weniger interessant. Die erfindungsgemäßen Katalysatoren zeichnen sich durch eine hohe Aktivität aus und können daher auch in geringen Mengen von 0,05 bis 3, bevorzugt 0,05 bis 2, besonders bevorzugt 0,1 bis 1,5 Gew.-%, bezogen auf den Alkohol, eingesetzt werden.

Die beiden Edukte, der Alkohol und das α,β-ungesättigte Nitril, werden üblicherweise in einem molaren Verhältnis derart eingesetzt, daß das molare Verhältnis von umzusetzender Hydroxylgruppe zu α,β-ungesättigtem Nitril im Bereich von 1:0,5 bis 1:10, bevorzugt 1:0,8 bis 1:2, besonders bevorzugt 1:0,9 bis 1:1,2, liegt. Setzt man weniger als 1 Mol α,β-ungesättigtes Nitril pro Mol umzusetzender Hydroxylgruppe ein, so erhält man einen unvollständigen Umsatz dieser Hydroxylgruppe und es liegt nach der Umsetzung so gut wie kein freies α,β-ungesättigtes Nitril mehr vor, was sicherheitstechnische Vorteile ergeben kann. Ein molarer Überschuß an α,β-ungesättigtem Nitril bezogen auf vorhandene Hydroxylgruppen bewirkt zwar einen vollständigen Umsatz des Alkohols, bringt jedoch dann Probleme bei der Aufarbeitung der Reaktionsanträge mit sich, da diese noch, zumeist toxische, unumgesetzte α,β-ungesättigte Nitrile enthalten.

Die erfindungsgemäßen Katalysatoren der Formel I zeichnen sich zusätzlich dadurch aus, daß sie auch bei molaren Verhältnissen von Hydroxylgruppe zu α,β-ungesättigtem Nitril im Bereich von 1:1 bis 1:1,1 noch einen vollständigen Alkoholumsatz bewirken, während weniger gut geeignete Anlagerungskatalysatoren einen größeren molaren Überschuß an α,β-ungesättigtem Nitril erfordern.

Sollen zwei- oder dreiwertige Alkohole an α,β-ungesättigte Nitrile addiert werden, so hat man es über die Steuerung der Reaktionsbedingungen, z. B. Wahl des molaren Verhältnisses von Alkohol zu α,β-ungesättigtem Nitril, in der Hand, Mono-, Bisoder Tris-1,4-Additionsprodukte zu erhalten. So kann z. B. durch die Umsetzung von Diethylenglykol mit einem Moläquivalent Acrylnitril das Produkt mit der Formel HO-(CH₂)₂-O-(CH₂)₂-O-(CH₂)₂-CN erhalten werden.

Als inerte Lösungsmittel für die Umsetzung eignen sich zum Beispiel Ether, wie Tetrahydrofuran (THF), Methyl-tert.-butylether (MTBE), 1,4-Dioxan, oder N-Methyl-pyrrolidon (NMP), N,N-Dimethylformamid (DMF), Dimethylsulfoxid (DMSO), Sulfolan, bevorzugt THF oder NMP. Auch Mischungen geeigneter Lösungsmittel sind verwendbar.

In vielen Fällen, wie in den Reaktionsbeispielen, kann die Umsetzung ohne Gegenwart eines Lösungsmittels durchgeführt werden.

Die Reaktionstemperatur richtet sich nach der Reaktivität des eingesetzten Alkohols bzw. α,β-ungesättigten Nitrils, den Schmelzpunkten sowie der Flüchtigkeit der Edukte. Geeignete Reaktionstemperaturen liegen in der Regel im Bereich von -20 bis +200 °C, wobei man u. U. die Umsetzung bei erhöhtem Druck vornimmt. Bevorzugt sind Reaktionstemperaturen von 0 bis 150 °C, besonders bevorzugt 25 bis 100 °C.

Wird die Umsetzung in Abwesenheit eines Lösungsmittels durchgeführt, so wird die Reaktionstemperatur mindestens so hoch eingestellt, daß der eingesetzte Alkohol flüssig vorliegt.

Der Reaktionsdruck (absolut gemessen) beträgt in der Regel 0,05 bis 2 MPa, bevorzugt 0,09 bis 1 MPa, besonders bevorzugt Atmosphärendruck (Normaldruck).

Bei der erfindungsgemäßen Umsetzung der Alkohole mit den α,β-ungesättigten Nitrilen werden in der Regel Verweilzeiten von 15 Min. bis 10 h eingestellt. Bevorzugt sind Verweilzeiten von 1 bis 5 h, besonders bevorzugt 1 bis 3 h. Je höher die gewählte Reaktionstemperatur, desto kürzer kann in der Regel die Verweilzeit bemessen sein.

Das erfindungsgemäße Verfahren eignet sich beispielsweise zur Herstellung von β-Alkoxy-nitrilen der Formel II 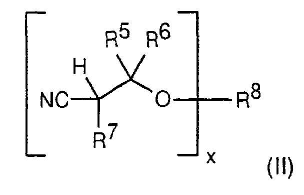 in der unabhängig voneinander
- R⁵, R⁶, R⁷: Wasserstoff, gegebenenfalls durch Halogen, Cyano, C₁₋₃₀-Alkoxy, C₆₋₃₀-Aryloxy, C₂₋₃-Alkenyloxy und/oder C₂₋₂₀-Dialkylamino substituiertes C₁₋₃₀-Alkyl, C₂₋₃₀-Alkenyl, C₃₋₁₂-Cycloalkyl, C₅₋₁₂-Cycloalkenyl, C₆₋₂₀-Aryl, C₃₋₁₅-Heteroaryl, C₇₋₂₀-Arylalkyl, C₈₋₂₀-Arylalkenyl, C₄₋₂₀-Heteroarylalkyl, C₇₋₂₀-Alkylaryl, C₄₋₂₀-Alkylheteroaryl, Y-(CH₂)ₐ-NR⁹-,
- R⁵ und R⁶: gemeinsam (CH₂)ₐ-X-(CH₂)_{b} oder gemeinsam eine durch Halogen, Cyano, C₁₋₃₀-Alkoxy, C₆₋₃₀-Aryloxy, C₂₋₃-Alkenyloxy und/oder C₂₋₂₀-Dialkylamino substituierte C₄₋₈-Alkylenkette,
- R⁵ und R⁷: gemeinsam (CH₂)ₐ-X-(CH₂)_{b} oder gemeinsam eine durch Halogen, Cyano, C₁₋₃₀-Alkoxy, C₆₋₃₀-Aryloxy, C₂₋₃-Alkenyloxy und/oder C₂₋₂₀-Dialkylamino substituierte C₄₋₈-Alkylenkette,
- X: CH₂, CHR⁹, O oder NR⁹,
- R⁹: C₁₋₄-Alkyl, C₆₋₂₀-Aryl, C₇₋₂₀-Alkylaryl, C₇₋₂₀-Arylalkyl,
- Y: C₃₋₃₀-Dialkylaminoalkyl,
- a, b: eine ganze Zahl von 1 bis 4,
wobei die Reste R⁵, R⁶ und R⁷ beispielsweise zusammen bis zu 37 C-Atome besitzen,
- x: eine ganze Zahl von 1 bis 3,
- R⁸: für den Fall, daß x = 1:
gegebenenfalls durch Halogen, Cyano, Hydroxy, C₁₋₃₀-Alkoxy, C₆₋₃₀-Aryloxy, C₂₋₃-Alkenyloxy und/oder C₂₋₂₀-Dialkylamino substituiertes C₁₋₂₀₀-Alkyl, C₃₋₂₀₀-Alkenyl, C₃₋₂₀₀-Alkinyl, C₃₋₁₂-Cycloalkyl, C₅₋₁₂-Cycloalkenyl, C₆₋₂₀-Aryl, C₃₋₁₅-Heteroaryl, C₇₋₂₀-Arylalkyl, C₈₋₂₀-Arylalkenyl, C₄₋₂₀-Heteroarylalkyl, C₇₋₂₀-Alkylaryl, C₄₋₂₀-Alkylheteroaryl oder Y-(CH₂)ₐ-NR⁹-(CH₂)_{b+1}-, CₙH₂ₙ₊₁-(NR⁹-C₁H₂₁)ₚ-NR⁹-C_{q}H_{2q}-, Alkoxyalkyl des Typs CₙH₂ₙ₊₁-O-CₘH₂ₘ-, Aryloxyalkyl des Typs Ar-O-CₘH₂ₘ-, mit Ar gleich C₆₋₂₀-Aryl,
Polyalkoxyalkyl des Typs CₙH₂ₙ₊₁-O-CₘH₂ₘ-(O-C₁H₂₁)ₚ-O-C_{q}H_{2q}-,
- m, n, l, q: eine ganze Zahl von 1 bis 20
- p: eine ganze Zahl von 0 bis 50
- R⁸: für den Fall, daß x = 2:
ein gegebenenfalls durch Halogen, Cyano, Hydroxy, C₁₋₃₀-Alkoxy, C₆₋₃₀-Aryloxy, C₂₋₃-Alkenyloxy und/oder C₂₋₂₀-Dialkylamino substituiertes/r
C₂₋₂₀₀-Alkan, C₄₋₂₀₀-Alken, C₄₋₂₀₀-Alkin, C₄₋₁₂-Cycloalkan, C₅₋₁₂-Cycloalken, C₆₋₂₀-Aromat, C₃₋₁₅-Heteroaromat, C₇₋₂₀-Alkylaromat, C₈₋₂₀-Alkenylaromat mit jeweils zwei freien Valenzen oder
-CₙH₂ₙ-(NR⁹-C₁H₂₁)ₚ-NR⁹-C_{q}H_{2q}-,
Polyoxypolyalkylen des Typs
-CₙH₂ₙ-O-CₘH₂ₘ-(O-C₁H₂₁)ₚ-O-C_{q}H_{2q}-,
- R⁸: für den Fall, daß x = 3:
ein gegebenenfalls durch Halogen, Cyano, C₁₋₃₀-Alkoxy, C₆₋₃₀-Aryloxy, C₂₋₃-Alkenyloxy und/oder C₂₋₂₀-Dialkylamino substituiertes/r
C₃₋₂₀₀-Alkan, C₅₋₂₀₀-Alken, C₅₋₁₂-Cycloalkan, C₅₋₁₂-Cycloalken, C₆₋₂₀-Aromat, C₇₋₂₀-Alkylaromat mit jeweils drei freien Valenzen oder
-CₙH₂ₙ₋₁-(NR⁹-C₁H₂₁)ₚ-NR⁹-C_{q}H_{2q}-,
Polyoxypolyalkylen des Typs
-CₙH₂ₙ₋₁-O-CₘH₂ₘ-(O-C₁H₂₁)ₚ-O-C_{q}H_{2q}-,
bedeuten,
durch Umsetzung von α,β-ungesättigten Nitrilen mit beispielsweise 3 bis 40 C-Atomen der Formel III 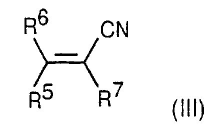 mit ein-, zwei- oder dreiwertigen Alkoholen, z. B. mit einer Molmasse von bis zu 2,5 • 10³ g/Mol, der Formel IV

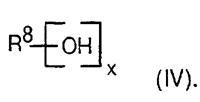

Die Reste R⁵, R⁶, R⁷, R⁸, R⁹ sowie die Bezeichnungen X, Y, a, b, x, m, n, l, q und p in den Verbindungen der Formeln II, III und IV haben unabhängig voneinander folgende Bedeutungen:
R⁵, R⁶, und R⁷:
   - gleich oder verschieden,
   - Wasserstoff,
   - C₁₋₃₀-Alkyl, vorzugsweise C₁₋₂₀-Alkyl, bevorzugt C₁₋₁₂-Alkyl, wie Methyl, Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Dimethylpropyl, n-Hexyl, iso-Hexyl, sec.-Hexyl, Cyclopropylmethyl, n-Heptyl, iso-Heptyl, Cyclohexylmethyl, n-Octyl, 2-Ethyl-hexyl, n-Nonyl, iso-Nonyl, n-Decyl, iso-Decyl, n-Undecyl, n-Dodecyl und iso-Dodecyl, besonders bevorzugt C₁₋₄-Alkyl, wie Methyl, Ethyl, n-Propyl, iso-Propyl-, n-Butyl-, iso-Butyl, sec.-Butyl-, tert.-Butyl,
   - C₂₋₂₀-Alkenyl, vorzugsweise C₂₋₂₀-Alkenyl, bevorzugt C₂₋₁₂-Alkenyl, wie Ethenyl, 2-Propen-1-yl, 2-Propen-2-yl, 2-Buten1-yl, 2-Buten-2-yl, 3-Buten-1-yl, 3-Buten-2-yl, 2-Penten-1-yl, 4-Penten-1-yl, 2-Hexen-1-yl, 5-Hexen-1-yl,
   - C₃₋₁₂-Cycloalkyl, vorzugsweise C₃₋₈-Cycloalkyl, wie Cyclopropyl, Cyclobutyl, Cyclopentyl, Cyclohexyl, Cycloheptyl und Cyclooctyl, bevorzugt Cyclopentyl, Cyclohexyl und Cyclooctyl, besonders bevorzugt Cyclopentyl und Cyclohexyl,
   - C₅₋₁₂-Cycloalkenyl, vorzugsweise C₅₋₈-Cycloalkenyl, wie 1-Cyclopentenyl, 3-Cyclopentenyl, 1-Cyclohexenyl, 3-Cyclohexenyl, 1-Cycloheptenyl und 1-Cyclooctenyl,
   - C₆₋₂₀-Aryl, wie Phenyl, 1-Naphthyl, 2-Naphthyl, 1-Anthryl, 2-Anthryl, 9-Anthryl bevorzugt Phenyl, 1-Naphthyl, 2-Naphthyl, besonders bevorzugt Phenyl,
   - C₃₋₁₅-Heteroaryl, wie 2-Pyridinyl, 3-Pyridinyl, 4-Pyridinyl, Pyrazinyl, Pyrrol-3-yl, Imidazol-2-yl, 2-Furanyl und 3-Furanyl,
   - C₇₋₂₀-Arylalkyl, bevorzugt C₇₋₁₂-Phenylalkyl, wie Benzyl, 1-Phenethyl, 2-Phenethyl, 4-tert.-Butyl-phenyl-methyl, 1-Phenylpropyl, 2-Phenylpropyl, 3-Phenylpropyl, 1-Phenylbutyl, 2-Phenylbutyl, 3-Phenylbutyl und 4-Phenylbutyl, besonders bevorzugt Benzyl, 1-Phenethyl und 2-Phenethyl,
   - C₈₋₂₀-Arylalkenyl, bevorzugt C₈₋₁₂-Phenylalkenyl, wie 1-Phenethenyl, 2-Phenethenyl, 3-Phenyl-1-propen-2-yl und 3-Phenyl-1-propen-1-yl,
   - C₄₋₂₀-Heteroarylalkyl, wie Pyrid-2-yl-methyl, Furan-2-yl-methyl, Pyrrol-3-yl-methyl und Imidazol-2-yl-methyl,
   - C₇₋₂₀-Alkylaryl, bevorzugt C₇₋₁₂-Alkylphenyl, wie 2-Methylphenyl, 3-Methylphenyl, 4-Methylphenyl, 2,4-Dimethylphenyl, 2,5-Dimethylphenyl, 2,6-Dimethylphenyl, 3,4-Dimethylphenyl, 3,5-Dimethylphenyl, 2,3,4-Trimethylphenyl, 2,3,5-Trimethylphenyl, 2,3,6-Trimethylphenyl, 2,4,6-Trimethylphenyl, 2-Ethylphenyl, 3-Ethylphenyl, 4-Ethylphenyl, 2-iso-Propylphenyl, 3-iso-Propylphenyl, 4-iso-Propylphenyl und 4-tert.-Butylphenyl,
   - C₄₋₂₀-Alkylheteroaryl, wie 2-Methyl-3-pyridinyl, 4-Methyl-imidazol-2-yl, 4,5-Dimethyl-imidazol-2-yl, 3-Methyl-2-furanyl und 5-Methyl-2-pyrazinyl,
   - Y-(CH₂)ₐ-NR⁹-, mit Y, a und R⁹ wie unten definiert, wie (CH₃)₂N-(CH₂)₂-NCH₃-, (CH₃)₂N-(CH₂)₃-NCH₃-, (CH₃)₂N-(CH₂)₄-NCH₃-,
R⁵ und R⁶:
   - gemeinsam (CH₂)ₐ-X-(CH₂)_{b}, mit a, b und X wie unten definiert, wie -(CH₂)₃-, -(CH₂)₄-, -(CH₂)₅-, -(CH₂)₆-, -(CH₂)₂-O-(CH₂)₂-, -CH₂-O-(CH₂)₂-, -CH₂-CHCH₃-(CH₂)₂-, -(CH₂)₂-NCH₃-(CH₂)₂-, -(CH₂)₂-N(CH₂Ph)-(CH₂)₂-,
R⁵ und R⁷:
   - gemeinsam (CH₂)ₐ-X-(CH₂)_{b}, mit a, b und X wie unten definiert, wie -(CH₂)₃-, -(CH₂)₄-, -(CH₂)₅-, -(CH₂)₆-, -(CH₂)₂-O-(CH₂)₂-, -CH₂-O-(CH₂)₂-, -CH₂-O-CH₂-, -CH₂-CHCH₃-(CH₂)₂-, -(CH₂)₂-NCH₃-(CH₂)₂-, -(CH₂)₂-N(CH₂Ph)-(CH₂)₂-,
R⁹:
   - C₁₋₄-Alkyl, wie Methyl, Ethyl, n-Propyl, iso-Propyl, n-Butyl, iso-Butyl, sec.-Butyl, tert.-Butyl,
   - C₆₋₂₀-Aryl, wie oben definiert,
   - C₇₋₂₀-Alkylaryl, wie oben definiert oder
   - C₇₋₂₀-Arylalkyl, wie oben definiert.
X:
   - CH₂, CHR⁹, O oder NR⁹
Y:
   - C₃₋₃₀-Dialkylaminoalkyl, bevorzugt C₃₋₂₀-Dialkylaminoalkyl, besonders bevorzugt C₃₋₁₂-Dialkylaminoalkyl, wie Dimethylaminomethyl, Diethylaminomethyl, Di-iso-propylaminomethyl, Di-npropylaminomethyl, 2-Dimethylaminoethyl, 2-Diethylaminoethyl, 2-Di-n-propylaminoethyl und 2-Di-iso-propylaminoethyl, (R⁹)₂N-(CH₂)ₐ,
a, b:
   - unabhängig voneinander,
   - eine ganze Zahl von 1 bis 4, bevorzugt 1, 2 oder 3,
   wobei die Reste R⁵, R⁶ und R⁷ beispielsweise zusammen bis zu 37 C-Atome besitzen,
x:
   eine ganze Zahl von 1 bis 3, bevorzugt 1 oder 2,
R⁸:
   für den Fall, daß x = 1:
   - C₁₋₂₀₀-Alkyl, vorzugsweise C₁₋₂₀-Alkyl, bevorzugt C₁₋₁₂-Alkyl, wie Methyl, Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 2-Methyl-but-2-yl, 1,2-Dimethylpropyl, n-Hexyl, iso-Hexyl, sec.-Hexyl, Cyclopentylmethyl, n-Heptyl, iso-Heptyl, Cyclohexylmethyl, n-Octyl, 2-Ethylhexyl, n-Nonyl, iso-Nonyl, n-Decyl, iso-Decyl, n-Undecyl, n-Dodecyl und iso-Dodecyl, besonders bevorzugt C₁₋₈-Alkyl, wie Methyl, Ethyl, n-Propyl, iso-Propyl-, n-Butyl, iso-Butyl, sec.-Butyl, tert.-Butyl, 2-Ethylhexyl,
      sowie bevorzugt C₄₀₋₂₀₀-Alkyl, wie Polybutyl, Polyisobutyl, Polypropyl, Polyisopropyl und Polyethyl, Polybutylmethyl, Polyisobutylmethyl, Polypropylmethyl, Polyisopropylmethyl und Polyethylmethyl,
   - C₃₋₂₀₀-Alkenyl, vorzugsweise C₃₋₂₀-Alkenyl, bevorzugt C₃₋₁₂-Alkenyl, wie 2-Propen-1-yl, 2-Buten-1-yl, 3-Buten-1-yl, 1-Buten-3-yl, 1-Buten-3-methyl-3-yl, 2-Penten-1-yl, 4-Penten-1-yl, 2-Hexen-1-yl, 5-Hexen-1-yl,
   - C₃₋₂₀₀-Alkinyl, vorzugsweise C₃₋₂₀-Alkinyl, bevorzugt C₃₋₁₂-Alkinyl, wie 2-Propin-1-yl, 2-Butin-1-yl, 3-Butin-1-yl, 1-Butin-3-yl, 1-Butin-3-methyl-3-yl, 2-Pentin-1-yl, 4-Pentin-1-yl, 2-Hexin-1-yl, 5-Hexin-1-yl, 1-Pentin-3-methyl-3-yl, 1-Octin-4-ethyl-3-yl,
   - C₃₋₁₂-Cycloalkyl, wie oben definiert,
   - C₅₋₁₂-Cycloalkenyl, wie oben definiert,
   - C₆₋₂₀-Aryl, wie oben definiert,
   - C₃₋₁₅-Heteroaryl, wie oben definiert,
   - C₇₋₂₀-Arylalkyl, wie oben definiert,
   - C₈₋₂₀-Arylalkenyl, wie oben definiert,
   - C₄₋₂₀-Heteroarylalkyl, wie oben definiert,
   - C₇₋₂₀-Alkylaryl, wie oben definiert,
   - C₄₋₂₀-Alkylheteroaryl, wie oben definiert,
   - Y-(CH₂)ₐ-NR⁹-(CH₂)_{b+1}-, mit Y, R⁹, a und b wie oben definiert, wie (CH₃)₂N-(CH₂)₂-NCH₃-(CH₂)₂-, (CH₃)₂N-(CH₂)₃-NCH₃-(CH₂)₃-,
   - CₙH₂ₙ₊₁-(NR⁹-C₁H₂₁)ₚ-NR⁹-C_{q}H_{2q}-, mit R⁹ wie oben definiert und n, l, p und q wie unten definiert, wie CH₃(CH₂)₂-(NCH₃-(CH₂)₂)₅-NCH₃-(CH₂)₃-, CH₃-(NCH₃-(CH₂)₂)₅-NCH₃-(CH₂)₂-,
   - Alkoxyalkyl des Typs CₙH₂ₙ₊₁-O-CₘH₂ₘ-, mit n und m wie unten definiert, wie CH₃OCH₂CH₂-, C₂H₅OCH₂CH₂-, CH₃O(CH₂)₄-,
   - Aryloxyalkyl des Typs Ar-O-CₘH₂ₘ-, mit Ar gleich C₆₋₂₀-Aryl wie oben definiert und m wie unten definiert, wie C₆H₅OCH₂CH₂-, C₆H₅O(CH₂)₄-,
   - Polyalkoxyalkyl des Typs CₙH₂ₙ₊₁-O-CₘH₂ₘ-(O-C₁H₂₁)ₚ-O-C_{q}H_{2q}-, mit n, m, l, p und q wie unten definiert, wie CH₃OCH₂CH₂(OCH₂CHCH₃)₃OCH₂CH₂-, CH₃OCH₂CH₂(OCH₂CH₂)₃OCH₂CH₂-,
m, n, l, q:
   - unabhängig voneinander,
   - eine ganze Zahl von 1 bis 20, bevorzugt von 1 bis 10,
p:
   eine ganze Zahl von 0 bis 50, bevorzugt von 0 bis 10,
R⁸:
   für den Fall, daß x = 2:
   - ein C₂₋₂₀₀-Alkan, vorzugsweise ein C₂₋₂₀-Alkan, bevorzugt ein C₂₋₁₂-Alkan, mit jeweils 2 freien Valenzen, wie Ethan-1,2-diyl, n-Propan-1,2-diyl, n-Propan-1,3-diyl, n-Butan-1,2-diyl, n-Butan-1,3-diyl, n-Butan-1,4-diyl, n-Butan-2,3-diyl, 2-Methyl-propan-1,3-diyl, n-Pentan-1,2-diyl, n-Pentan-1,5-diyl, 2-Methyl-butan-1,4-diyl, 2,2-Dimethyl-propan-1,3-diyl, 1,2-Dimethylpropan-1,3-diyl, n-Hexan-1,2-diyl, n-Hexan-1,6-diyl, n-Hexan-2,5-diyl, n-Heptan-1,2-diyl, n-Heptan-1,7-diyl, n-Octan-1,2-diyl, n-Octan-1,8-diyl, 2,2,4-Trimethyl-pentan-1,3-diyl, n-Nonan-1,2-diyl, n-Decan-1,2-diyl, n-Decan-1,10-diyl, n-Undecan-1,2-diyl, n-Dodecan-1,2-diyl, besonders bevorzugt ein C₂₋₄-Alkan mit jeweils 2 freien Valenzen, wie Ethan-1,2-diyl, n-Propan-1,2-diyl, n-Propan-1,3-diyl, n-Butan-1,2-diyl, n-Butan-1,4-diyl, 2,2-Dimethyl-propan-1,3-diyl, n-Hexan-1,6-diyl,
      sowie vorzugsweise ein C₄₀₋₂₀₀-Alkan, wie Polybutan, Polyisobutan, Polypropan, Polyisopropan und Polyethan, Polybutylmethan, Polyisobutylmethan, Polypropylmethan, Polyisopropylmethan und Polyethylmethan mit jeweils 2 freien Valenzen,
   - ein C₄₋₂₀₀-Alken, vorzugsweise ein C₄₋₂₀-Alken, bevorzugt ein C₄₋₁₂-Alken, mit jeweils 2 freien Valenzen, wie cis-2-Buten-1,4-diyl, trans-2-Buten-1,4-diyl, 3-Buten-1,2-diyl, 2-Penten-1,4-diyl, 4-Penten-1,2-diyl, 2-Hexen-1,6-diyl, 3-Hexen-1,6-diyl, 3-Hexen-2,5-diyl, 5-Hexen-1,2-yl, 2,5-Dimethyl-3-hexen-2,5-diyl,
   - ein C₄₋₂₀₀-Alkin, vorzugsweise ein C₄₋₂₀-Alkin, bevorzugt ein C₄₋₁₂-Alkin, mit jeweils 2 freien Valenzen, wie 1-Butin-3,4-diyl, 2-Butin-1,4-diyl, 3-Hexin-1,6-diyl, 3-Hexin-2, 5-diyl, 2,5-Dimethyl-3-hexin-2,5-diyl, 1-Hexin-5,6-diyl,
   - ein C₄₋₁₂-Cycloalkan, vorzugsweise ein C₄₋₈-Cycloalkan, mit jeweils 2 freien Valenzen, wie Cyclobutan-1,2-diyl, Cyclopentan-1,2-diyl, Cyclopentan-1,3-diyl, Cyclohexan-1,2-diyl, Cyclohexan-1,3-diyl, Cyclohexan-1,4-diyl, Cycloheptan-1,2-diyl und Cyclooctan-1,2-diyl, bevorzugt Cyclopentan-1,2-diyl, Cyclohexan-1,2-diyl, Cyclohexan-1,3-diyl, Cyclohexan-1,4-diyl und Cyclooctan-1,2-diyl, besonders bevorzugt Cyclopentan-1,3-diyl und Cyclohexan-1,2-diyl,
   - ein C₅₋₁₂-Cycloalken, vorzugsweise ein C₅₋₈-Cycloalken, mit jeweils 2 freien Valenzen, wie 1-Cyclopenten-3,4-diyl, 1-Cyclopenten-3,5-diyl, 1-Cyclohexen-3,4-diyl, 1-Cyclohexen-3,6-diyl, 1-Cyclohepten-3,4-diyl, 1-Cyclohepten-3,7-diyl und 1-Cycloocten-3,8-diyl,
   - C₆₋₂₀-Aromat mit jeweils 2 freien Valenzen, wie Benzol-1,2-diyl, Benzol-1,3-diyl, Benzol-1,4-diyl, Naphthalin-1,2-diyl, Naphthalin-1,4-diyl, Naphthalin-1,5-diyl, Naphthalin-1,8-diyl, Anthracen-1,2-diyl, Anthracen-1,3-diyl, Anthracen-1,5-diyl,
   - C₃₋₁₅-Heteroaromat mit jeweils 2 freien Valenzen, wie Pyridin-2,3-diyl, Pyridin-2,4-diyl, Pyrazin-2,3-diyl, Pyrrol-2,3-diyl, Imidazol-4,5-diyl, Furan-2,3-diyl und Furan-3,4-diyl,
   - C₇₋₂₀-Alkylaromat mit jeweils 2 freien Valenzen, wie Benzyl-2-yl, Benzyl-4-yl, 1-Methyl-benzol-3,4-diyl, 1-Phenylpropan-2,3-diyl, 2-Phenylpropan-1,3-diyl, 3-Phenylbutan-1,2-diyl,
   - C₈₋₂₀-Alkenylaromat mit jeweils 2 freien Valenzen, wie 1-Vinyl-benzol-3,4-diyl,
   - -CₙH₂ₙ-(NR⁹-C₁H₂₁)ₚ-NR⁹-C_{q}H_{2q}-, mit R⁹, n, l, p und q wie oben definiert, wie -CH₂CH₂(N(CH₃)CH₂CH₂)₄-NCH₃-CH₂CH₂-,
   - Polyoxypolyalkylen des Typs -CₙH₂ₙ-O-CₘH₂ₘ-(O-C₁H₂₁)ₚ-O-C_{q}H_{2q}-, mit n, m, l, p und q wie oben definiert, wie zum Beispiel - (CH₂)₄-(O-CH₂CH₂CH₂CH₂)₂₆-, -CH₂CH₂(OCH₂CH₂)₂-,
R⁸:
   für den Fall, daß x = 3:
   - ein C₃₋₂₀₀-Alkan, vorzugsweise ein C₂₋₂₀-Alkan, bevorzugt ein C₂₋₁₂-Alkan, mit jeweils 3 freien Valenzen, wie n-Propan-1,2,3-triyl, n-Butan-1,2,3-triyl, n-Butan-1,3,4-triyl, 2-Methyl-propan-1,2,3-triyl, n-Pentan-1,2,3-triyl, n-Pentan-1,4,5-triyl, 2-Methyl-butan-1,3,4-triyl, 1,2-Dimethylpropan-1,2,3-triyl, n-Heptan-1,2,3-triyl, n-Hexan-1,2,6-triyl, n-Hexan-2,3,5-triyl, n-Heptan-1,2,3-triyl, n-Heptan-1,6,7-triyl, n-Octan-1,2,3-triyl, n-Octan-1,7,8-triyl, n-Nonan-1,2,3-triyl, n-Decan-1,2,3-triyl, n-Decan-1,9,10-triyl, n-Undecan-2,2,3-tiyl, n-Dodecan-1,2,3-triyl, besonders bevorzugt ein C₃₋₅-Alkan mit jeweils 3 freien Valenzen, wie n-Propan-1,2,3-triyl, n-Butan-1,2,3-triyl, n-Butan-1,3,4-triyl,
   - ein C₅₋₂₀₀-Alken, vorzugsweise ein C₅₋₂₀-Alken, bevorzugt ein C₅₋₁₂-Alken, mit jeweils 3 freien Valenzen, wie 2-Penten-1,4,5-triyl, 2-Hexen-1,5,6-triyl,
   - ein C₅₋₁₂-Cycloalkan, vorzugsweise ein C₅₋₈-Cycloalkan, mit jeweils 3 freien Valenzen, wie Cyclopentan-1,2,3-triyl, Cyclopentan-1,3,4-triyl, Cyclohexan-1,2,3-triyl, Cyclohexan-1,3,4-triyl, Cyclohexan-1,2,5-triyl,
   - ein C₅₋₁₂-Cycloalken, vorzugsweise ein C₅₋₈-Cycloalken, mit jeweils 3 freien Valenzen, wie 1-Cyclopenten-3,4,5-triyl, 1-Cyclohexen-3,4,5-triyl, 1-Cyclohexen-3,4,6-triyl,
   - C₆₋₂₀-Aromat mit jeweils 3 freien Valenzen, wie Benzol-1,2,3-triyl, Benzol-1,3,4-triyl, Naphthalin-1,2,8-triyl, Naphthalin-1,3,8-triyl, Anthracen-1,2,5-triyl,
   - C₇₋₂₀-Alkylaromat mit jeweils 3 freien Valenzen, wie Benzyl-2,3-diyl, Benzyl-3,4-diyl, 1-Methyl-benzol-3,4,5-triyl, 1-Phenylpropan-1,2,3-diyl, 2-Phenylpropan-1,2,3-triyl,
   - -CₙH₂ₙ₋₁-(NR⁹-C₁H₂₁)ₚ-NR⁹-C_{q}H_{2q}-, mit R⁹, n, l, p und q wie oben definiert, wie -CH₂CH(-)-N(CH₃)CH₂CH₂N(CH₃)CH₂CH₂-,
   - Polyoxypolyalkylen des Typs -CₙH₂ₙ₋₁-O-CₘH₂ₘ-(O-C₁H₂₁)ₚ-O-C_{q}H_{2q}-, mit n, m, l, p und q wie oben definiert, wie -CH₂CH(-)-(OCH₂CH₂)₃-O-CH₂CH₂CH₂-, -CH₂CH(-)-(OCH₂CH₂)₃-O-CH₂CH₂-, -CH₂CH(-)CH₂-(OCH₂CH₂)₃-O-CH₂CH₂-.

Die Reste R⁵, R⁶, R⁷ und R⁸ können wie oben angegeben substituiert sein. Dabei kann die Anzahl der Substituenten in Abhängigkeit von der Art des Restes 0 bis 5, vorzugsweise 0 bis 3, insbesondere 0, 1 oder 2 betragen. Als Substituenten kommen in Betracht:
- Halogen, wie Fluor, Chlor, Brom oder Iod, vorzugsweise Fluor oder Chlor,
- Cyano: -C≡N,
- C₁₋₃₀-Alkoxy, bevorzugt C₁₋₈-Alkoxy, wie Methoxy, Ethoxy, n-Propoxy, iso-Propoxy, n-Butoxy, iso-Butoxy, sec.-Butoxy, tert.-Butoxy, n-Pentoxy, iso-Pentoxy, sec.-Pentoxy, neo-Pentoxy, 1,2-Dimethylpropoxy, n-Hexoxy, iso-Hexoxy, sec.-Hexoxy, n-Heptoxy, iso-Heptoxy, n-Octoxy, iso-Octoxy, besonders bevorzugt C₁₋₄-Alkoxy, wie Methoxy, Ethoxy, n-Propoxy, iso-Propoxy, n-Butoxy, iso-Butoxy, sec.-Butoxy und tert.-Butoxy,
- C₆₋₃₀-Aryloxy, wie Phenoxy, 1-Naphthoxy und 2-Naphthoxy, bevorzugt Phenoxy,
- C₂₋₃-Alkenyloxy, wie Vinyloxy, 1-Propenyloxy,
- C₂₋₂₀-Dialkylamino, bevorzugt C₂₋₁₂-Dialkylamino, besonders C₂₋₈-Dialkylamino, wie N,N-Dimethylamino, N,N-Diethylamino, N,N-Dipropylamino, N,N-Di-(1-methylethyl)amino, N,N-Dibutylamino, N,N-Di-(1-methylpropyl)-amino, N,N-Di- (2-methylpropyl)amino, N,N-Di-(1,1-dimethylethyl)-amino, N-Ethyl-N-methylamino, N-Methyl-N-propylamino, N-Methyl-N-(1-methylethyl)-amino, N-Butyl-N-methylamino, N-Methyl-N-(1-methylpropyl)amino, N-Methyl-N-(2-methylpropyl)amino, N-(1,1-Dimethylethyl)-N-methylamino, N-Ethyl-N-propylamino, N-Ethyl-N-(1-methyl-ethyl)-amino, N-Butyl-N-ethylamino, N-Ethyl-N-(1-methylpropyl)amino,N-Ethyl-N-(2-methylpropyl)amino, N-Ethyl-N-(1,1-dimethylethyl)amino, N-(1-Methylethyl)-N-propylamino, N-Butyl-N-propylamino, N-(1-Methyl-propyl)-N-propylamino, N-(1-Methylpropyl)-N-propylamino, N-(2-Methyl-propyl)-N-propylamino, N-(1,1-Dimethylethyl)-N-propylamino, N-Butyl-N-(1-methylethyl)amino, n-(1-Methylethyl)-N-(1-methylpropyl)amino, N-(1-Methylethyl)-N-(1-methpropyl)amino, N-(1-Methylethyl)-N-(2-methylpro-pyl) amino, N-(1,1-Dimethylethyl)-N-(1-methylethyl)amino, N-Butyl-N-(1-methylpropyl) amino, N-Butyl-N- (2-methylpropyl)amino, N-Butyl-N-(1,1-dimethylethyl)amino, N-(1-Methylpropyl)-N-(2-methylpropyl)amino, N-(1,1-Dimethylethyl)-N-(1-methylpropyl)amino und N-(1,1-Dimethylethyl)-N-(2-methylpropyl)amino, Diallylamino, Dicyclohexylamino,

Als erfindungsgemäß einsetzbare Nitrile eignen sich eine Vielzahl aliphatischer oder aromatischer α,β-ungesättigter Nitrile. Die α,β-ungesättigten Nitrile können geradkettig oder verzweigt sein, alicyclische oder heterocyclische Gruppen enthalten und Halogen-, Cyano-, Alkoxy-, Aryloxy-, Alkenyloxy- und/oder Dialkylamino-Gruppen als Substituenten tragen.

Die Zahl der C-Atome der α,β-ungesättigten Nitrile ist nicht kritisch; üblicherweise werden niedermolekulare α,β-ungesättigte Nitrile mit bis zu 40 C-Atomen verwendet.

Beispiele für α,β-ungesättigte Nitrile der Formel III sind:

Acrylnitril, Methacrylnitril, Crotonnitril, 2-Pentennitril, β,β-Dimethyl-acrylnitril, β-Phenyl-acrylnitril. Bevorzugt sind aliphatische α,β-ungesättigte Nitrile mit bis zu 5 C-Atomen. Besonders bevorzugt ist Acrylnitril.

Als erfindungsgemäß einsetzbare Alkohole eignen sich eine Vielzahl aliphatischer oder aromatischer, ein-, zwei- oder dreiwertiger Alkohole. Die Alkohole können geradkettig oder verzweigt sein, alicyclische oder heterocyclische Gruppen enthalten und Halogen-, Cyano-, Alkoxy-, Aryloxy-, Alkenyloxy- und/oder Dialkylamino-Gruppen als Substituenten tragen. Die Reaktionsgeschwindigkeit von tertiären Alkoholen ist in der Regel geringer als die von primären oder sekundären Alkoholen. Die Molmasse der Alkohole beträgt beispielsweise bis zu 2,5 • 10³ g/Mol.

Beispiele für Alkohole der Formel IV sind:

Monoole, z. B. aliphatische Alkanole, wie Methanol, Ethanol, n-Propanol, Isopropanol, n-Butanol, Isobutanol, tert.-Butanol, n-Pentanol, 2-Methyl-2-butanol, n-Hexanol, Cyclopentanol, Cyclohexanol, 3,4-Dimethyl-cyclohexanol, 2-Ethylhexanol, 1-Octanol, Dodekanol, Tridecanol, Octadekanol, Menthol, Geraniol, Linalool, Citronellol, Stearylalkohol, Palmitylalkohol, Kokosalkohol, Oleylalkohol, Fettalkohole, Allylalkohol, Methallylalkohol, 4-Vinyloxy-1-butanol, Propargylalkohol, n-Amylalkohol, 1-Buten-3-ol, 1-Butin-3-ol, 1-Butin-3-methyl-3-ol, 1-Pentin-3-methyl-3-ol, 1-Octin-4-ethyl-3-ol, Polyisobutylalkohole, Polypropylalkohole, aromatische Alkohole, wie Phenol, Kresole, α- oder β-Naphthol, Benzylalkohol, Zimtalkohol, 4-tert.-Butyl-benzylalkohol, Furfurylalkohol, Alkoxy- oder Aryloxyalkohole, wie 2-Methoxyethanol, 2-Ethoxyethanol, 2-Propoxyethanol, 2-Butoxyethanol, 2-Phenoxyethanol, Cyanoalkohole, wie Formaldehydcyanhydrin, β-Cyanoethanol, Aminoalkohole, wie Dimethylaminoethanol, Diethylaminoethanol, 1-Dimethylamino-4-pentanol, 1-Diethylamino-4-pentanol, β-Morpholinoethanol, Triethanolamin, oder die durch Anlagerung von Alkylenoxiden wie Ethylenoxid, Propylenoxid oder Isobutylenoxid an Alkohole, wie z. B. Methanol, Ethanol, 1-Butanol, entstehenden Polyetheralkohole, wie Methylhexaglykol oder Butyltriglykol.

Bevorzugt sind Alkan-monoole und Alkoxyalkan-monoole mit 1 bis 12 C-Atomen, wie Methanol, Ethanol, 2-Ethylhexanol, 2-Methoxyethanol und Benzylalkohol.

Diole, z. B. Alkandiole, wie Ethylenglykol, 1,2-Propylenglykol, 1,3-Propylenglykol, 2,2-Dimethyl-1,3-propandiol (Neopentylglykol), 1,4-Butandiol, 2,3-Butandiol, 1,5-Pentandiol, 1,6-Hexandiol, 2,5-Hexandiol, 2,2,4-Trimethyl-pentan-1,3-diol, 1,4-Cyclohexandimethanol, 2,2-Bis[4-hydroxycyclohexyl]propan, Di-β-hydroxyethylether, 2-Butin-1,4-diol, 2-Buten-1,4-diol, 3-Hexin-1,6-diol, 3-Hexin-2,5-diol, 2,5-Dimethyl-3-hexin-2,5-diol, Oligo- oder Polyalkylenglykole, wie Diethylenglykol, Triethylenglykol, Polyethylenglykol, Polypropylenglykol oder Polytetrahydrofuran, aromatische Alkohole, wie Hydrochinon, oder andere Diole, wie z. B. Hydroxypivalinsäure-neopentylglykolmonoester.

Bevorzugt sind Ethylenglykol, Diethylenglykol, 1,4-Butandiol und Polytetrahydrofuran.

Triole, z. B. Alkantriole, wie Glycerin, Trimethylolpropan, Trimethylolethan, oder die durch Anlagerung von Alkylenoxiden wie Ethylenoxid oder Propylenoxid an Triole entstehenden Produkte.

Die 1,4-Additionsprodukte der Formel II können aus den durch die erfindungsgemäße Umsetzung von α,β-ungesättigten Nitrilen der Formel III mit Alkoholen der Formel IV erhaltenen Reaktionsausträgen in üblicher Weise, beispielsweise durch fraktionierende Rektifikation oder Kristallisation, erhalten werden. Dabei ist in der Regel eine zuvor erfolgende Abtrennung oder Neutralisation des Katalysators der Formel I, die zum Beispiel mit Säuren wie Ameisensäure, Essigsäure, 2-Ethylhexansäure, Salzsäure, p-Toluolsulfonsäure oder Phosphorsäure erfolgen könnte, nicht notwendig.

Es wurde weiterhin gefunden, daß die durch die erfindungsgemäße Umsetzung von α,β-ungesättigten Nitrilen der Formel III mit Alkoholen der Formel IV erhaltenen Reaktionsausträge, die die 1,4-Additionsprodukte der Formel II enthalten, direkt in einem zweiten Verfahrensschritt einer Hydrierung in Gegenwart eines Hydrierkatalysators unterworfen werden können, wobei γ-Alkoxyamine der Formel V 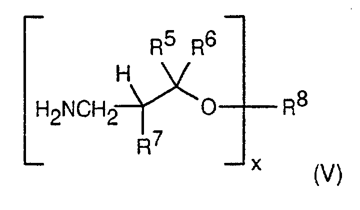 erhalten werden, ohne daß eine vorherige aufwendige Abtrennung oder Neutralisation des 1,4-Additions-Katalysators der Formel I notwendig ist. Die Hydrierung der β-Alkoxy-nitrile II in Gegenwart eines Hydrierkatalysators kann also in Gegenwart der Diazabicycloalkene I durchgeführt werden, ohne daß damit ein Nachteil verbunden ist.

Unter den für die β-Alkoxy-nitrile II üblichen Hydrierbedingungen werden die Diazabicycloalkene I nicht hydriert, sie finden sich beispielsweise für den Fall, daß sie schwerflüchtiger als das Amin V sind, nach einer Rektifikation des Amins V im Sumpf komplett wieder, von wo aus sie sogar wiedergewonnen werden können, um ein Recycling des Katalysators zu ermöglichen.

Für die Hydrierung der β-Alkoxy-nitrile II können alle für diese Reaktion gängigen Hydrierkatalysatoren verwendet werden, wie beispielsweise Raney-Nickel oder Raney-Cobalt, Nickel- oder Cobalt-Festbettkatalysatoren, Nickel oder Cobalt auf Trägern und edelmetallhaltige Trägerkatalysatoren. Der Hydrierkatalysator kann pulverförmig, als Suspension oder als Formkörper, wie Stränge, Tabletten oder Kugeln, eingesetzt werden.

Die Reaktionsbedingungen richten sich u. a. nach dem verwendeten Hydrierkatalysator und dem eingesetzten β-Alkoxy-nitril II. In der Regel wird die Hydrierung der β-Alkoxy-nitrile II, gegebenenfalls in Gegenwart eines inerten Lösungsmittels, wie z. B. 1,4-Dioxan, NMP, Tetrahydrofuran, Benzol, Toluol, Xylol, Methanol, Ethanol oder Butanol, bei Temperaturen von 50 bis 250 °C, Drücken von 3 bis 35 MPa und Verweilzeiten von 20 Min. bis 100 h durchgeführt.

Die Hydrierung kann sowohl diskontinuierlich, z. B. in Rühr-Autoklaven, als auch kontinuierlich, z. B. in Rohrreaktoren, durchgeführt werden.

Geeignete Ausführungsbeispiele für die Hydrierung der β-Alkoxynitrile II finden sich z. B. in Houben-Weyl, Methoden der organischen Chemie, Band 11/1, Seite 341 ff, 4. Auflage (1957), US 5,196,589 oder W. P. Utermohlen, J. Am. Chem. Soc. 67, 1505-6 (1945).

Besonders bevorzugt wird die Hydrierung der β-Alkoxy-nitrile II, die die Diazabicycloalkene I enthalten, bei Temperaturen von 70 bis 200 °C, Drücken von 5 bis 25 MPa und Verweilzeiten von 30 Min. bis 10 h ohne Gegenwart eines Lösungsmittels kontinuierlich durchgeführt.

β-Alkoxy-nitrile sind wichtige Zwischenprodukte zur Synthese von Wirk- und Farbstoffen.

γ-Alkoxy-amine sind als Vorprodukte für die Bildung von Kunststoffen, wie zum Beispiel Polyurethanen, Polyamiden und Epoxidharzen von erheblicher technischer Bedeutung.

### Beispiele

Die Messungen der APHA-Farbzahlen erfolgten nach DIN-ISO 6271.

### Beispiel 1: Methanol

52,48 g Methanol (1,64 mol) und 0,52 g DBU wurden vorgelegt und bei 55 °C langsam (5 %/Min.) 80,56 g Acrylnitril (ACN) (1,52 mol) hinzugefügt. Die Mischung wurde noch zwei Stunden bei 55 °C nachreagieren gelassen.

Die gaschromatographisch im Rohaustrag bestimmte Ausbeute an 3-Methoxypropionitril betrug 94 %, der Gehalt an freiem Acrylnitril nach Titration 0,7 % und die Farbzahl des Rohaustrags 3 APHA.

### Vergleichsbeispiel 1: Methanol

Analog zu Beispiel 1 wurde die Reaktion mit 0,52 g einer 50 %igen wäßrigen Lösung von Tetrakis-(2-hydroxyethyl)-ammoniumhydroxid durchgeführt.

Die gaschromatographisch im Rohaustrag bestimmte Ausbeute an 3-Methoxypropionitril betrug 94 %, der Gehalt an freiem Acrylnitril nach Titration 0,4 % und die Farbzahl des Rohaustrags 37 APHA.

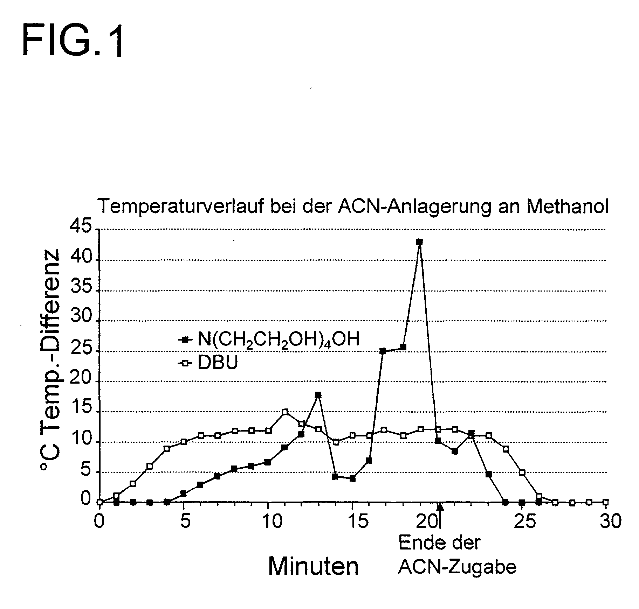

Aus dem in Diagramm 1 dargestellten Temperaturverlauf wird ersichtlich, daß in Beispiel 1 mit DBU als Katalysator eine sehr gleichmäßige Temperaturentwicklung während der Zugabe (ΔTₘₐₓ = 15 °C) vorlag, während der Vergleichsversuch 1 eine Temperaturspitze von 43 °C aufwies, die erst kurz vor Beendigung der ACN-Zugabe erreicht wurde. Die Reaktion sprang mit Tetrakis-(2-hydroxyethyl)-ammoniumhydroxid als Katalysator erst sehr verspätet an, was ein großes Sicherheitsrisiko bei Umsetzungen im technischen Maßstab darstellt.

### Beispiel 2: Diethylenglykol

89,88 g Diethylenglykol (0,85 mol) und 0,90 g DBU wurden vorgelegt und bei 55 °C langsam (5 %/Min.) 80,56 g Acrylnitril (1,52 mol) hinzugefügt. Die Mischung wurde noch zwei Stunden bei 55 °C nachreagieren gelassen.

Die gaschromatographisch im Rohaustrag bestimmte Ausbeute an Bis-cyanethyliertem Diethylenglykol betrug 85,0 %, die an mono-cyanethyliertem Diethylenglykol betrug 13,3 %, der Gehalt an freiem Acrylnitril nach Titration 0,77 % und die Farbzahl des Rohaustrags < 10 APHA.

### Vergleichsbeispiel 2: Diethylenglykol

Analog zu Beispiel 2 wurde die Reaktion mit 0,85 g einer 50 %igen wäßrigen Lösung von Tetrakis-(2-hydroxyethyl)-ammoniumhydroxid durchgeführt.

Die gaschromatographisch im Rohaustrag bestimmte Ausbeute an Bis-cyanethyliertem Diethylenglykol betrug 82,5 %, die an mono-cyanethyliertem Diethylenglykol betrug 14,8 %, der Gehalt an freiem Acrylnitril nach Titration 1,35 % und die Farbzahl des Rohaustrags 187 APHA.

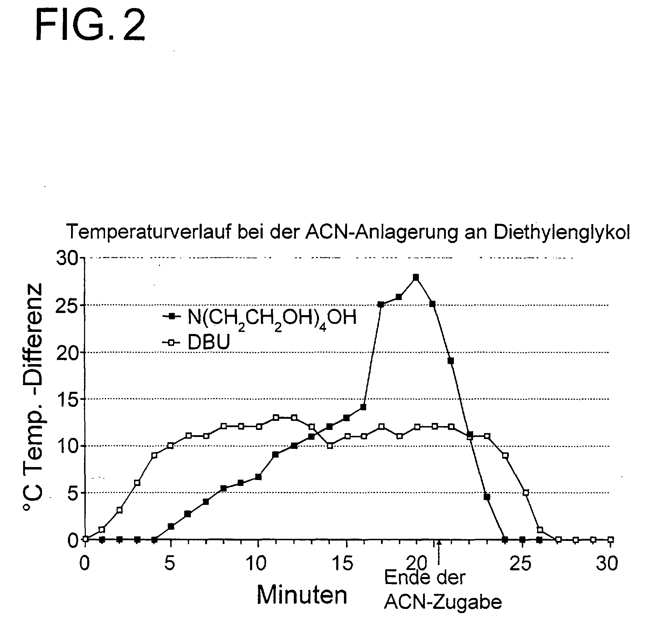

Aus dem in Diagramm 2 dargestellten Temperaturverlauf wird ersichtlich, daß in Beispiel 2 mit DBU eine sehr gleichmäßige Temperaturentwicklung während der Zugabe (ΔTₘₐₓ = 13 °C) vorlag, während der Vergleichsversuch 2 eine Temperaturspitze von 27 °C aufwies, die erst kurz vor Beendigung der ACN-Zugabe erreicht wurde. Die Reaktion sprang mit dem Tetrakis-(2-hydroxyethyl)-ammoniumhydroxid erst sehr verspätet an, was ein großes Sicherheitsrisiko bei Umsetzungen im technischen Maßstab darstellt.

### Beispiel 3: Diethylenglykol

85,24 g Diethylenglykol (0,80 mol) und 0,85 g DBU wurden vorgelegt und bei 55 °C langsam (5 %/Min.) 80,56 g Acrylnitril (1,52 mol) hinzugefügt. Die Mischung wurde noch zwei Stunden bei 55 °C nachreagieren gelassen.

Die gaschromatographisch im Rohaustrag bestimmte Ausbeute an Bis-cyanethyliertem Diethylenglykol betrug 90,7 %, die an mono-cyanethyliertem Diethylenglykol betrug 7,8 %, der Gehalt an freiem Acrylnitril nach Titration 0,33 %.

### Vergleichsbeispiel 3: Diethylenglykol

Analog zu Beispiel 3 wurde die Reaktion mit 0,90 g einer 50 %igen wäßrigen Lösung Tetrakis-(2-Hydroxyethyl)-ammoniumhydroxid durchgeführt.

Die gaschromatographisch im Rohaustrag bestimmte Ausbeute an Bis-cyanethyliertem Diethylenglykol betrug 90,0 %, die an mono-cyanethyliertem Diethylenglykol betrug 8,9 %, der Gehalt an freiem Acrylnitril nach Titration 0,80 %.

### Beispiel 4: Benzylalkohol

88,67 g Benzylalkohol (0,82 mol) und 0,88 g DBU wurden vorgelegt und bei 55 °C langsam (5 %/Min.) 40,30 g Acrylnitril (0,76 mol) hinzugefügt. Die Mischung wurde noch zwei Stunden bei 55 °C nachreagieren gelassen.

Die gaschromatographisch im Rohaustrag bestimmte Ausbeute an 3-Benzyloxypropionitril betrug 87,9 %, der Gehalt an freiem Acrylnitril nach Titration 2,24 % und die Farbzahl des Rohaustrags 3 APHA.

### Vergleichsbeispiel 4: Benzylalkohol

152,2 g Benzylalkohol (1,41 mol) und 0,71 ml einer 40 %igen methanolischen Lösung von Triton B (Benzyltrimethyl-ammoniumhydroxid) wurden vorgelegt und bei 45 °C langsam (5 %/Min.) 61,3 g Acrylnitril (1,16 mol) hinzugefügt. Die Mischung wurde noch drei Stunden bei 45 °C nachreagieren gelassen. Nach Beendigung der Reaktion wurde mit 1,2 ml Eisessig auf pH 6,4 eingestellt.

Die gaschromatographisch im Rohaustrag bestimmte Ausbeute an 3-Benzyloxypropionitril betrug 83,1 %, der Gehalt an freiem Acrylnitril nach Titration 0,34 % und die Farbzahl des Rohaustrags 90 APHA.

### Beispiel 5: Diethylenglykol

60,0 kg Diethylenglykol (566 mol) und 600 g DBU wurden vorgelegt und bei 55 °C langsam (5 %/Min.) 58,4 kg Acrylnitril (1102 mol) hinzugefügt. Die Mischung wurde noch zwei Stunden bei 55 °C nachreagieren gelassen. Das cyanethylierte Diethylenglykol wurde dann bei 110 °C an einem Kobalt-Katalysator kontinuierlich hydriert, die Austräge destilliert und analysiert. Eingesetzt wurden 250 ml Katalysator, die bei 20 MPa Wasserstoffdruck mit 75 g h⁻¹ Bis-cyanoethyl-diethylenglykol und 330 g h⁻¹ NH₃ beaufschlagt wurden. Die Katalysatorbelastung betrug 0,3 1_{Edukt}1_{Kat.}⁻¹h⁻¹.

Die Ausbeute an 4,7,10-Trioxatridecan-1,13-diamin (TTD) betrug über einen Zeitraum von 14 Tagen konstant 88 %, die an 1,4,7-Trioxa-undecan-11-amin betrug 8 %, sonstige Produkte und Destillationsrückstand lagen bei 4 %.

### Vergleichsbeispiel 5: Diethylenglykol

Analog zu Beispiel 5 wurde die Anlagerung mit 600 g einer 50 %igen wäßrigen Lösung von Tetrakis-(2-hydroxyethyl)-ammoniumhydroxid durchgeführt.

Die Ausbeute an 4,7,10-Trioxatridecan-1,13-diamin (TTD) betrug über einen Zeitraum von 21 Tagen konstant 75 %, die an 1,4,7-Trioxa-undecan-11-amin betrug 17 %, sonstige Produkte und Destillationsrückstand lagen bei 8 %.

### Beispiel 6: Diethylenglykol

Jeweils 100,00 g Diethylenglykol (0,94 mol) wurden mit 0,2, 0,4, 0,6, 0,8 und 1,0 % DBN vorgelegt und bei 55 °C langsam (5 %/Min.) 80,56 g Acrylnitril (1,52 mol) hinzugefügt. Die Mischung wurde noch zwei Stunden bei 55 °C nachreagieren gelassen.

Die gaschromatographisch im Rohaustrag bestimmten Ausbeuten an Bis-cyanethyliertem Diethylenglykol (BCE-DG), an mono-cyanethyliertem Diethylenglykol (MCE-DG) und an freiem Acrylnitril (ACN) sind in Tabelle 1 zusammengestellt.

**Tabelle 1**

| Gew.-% DBN | Ausbeute BCE-DG [%] | Ausbeute MCE-DG [%] | % ACN |
|---|---|---|---|
| 0,2 | 73,4 | 21,8 | 4,0 |
| 0,4 | 75,6 | 19,9 | 4,0 |
| 0,6 | 77,0 | 18,2 | 3,4 |
| 0,8 | 78,7 | 17,1 | 2,9 |
| 1,0 | 79,8 | 16,5 | 2,7 |

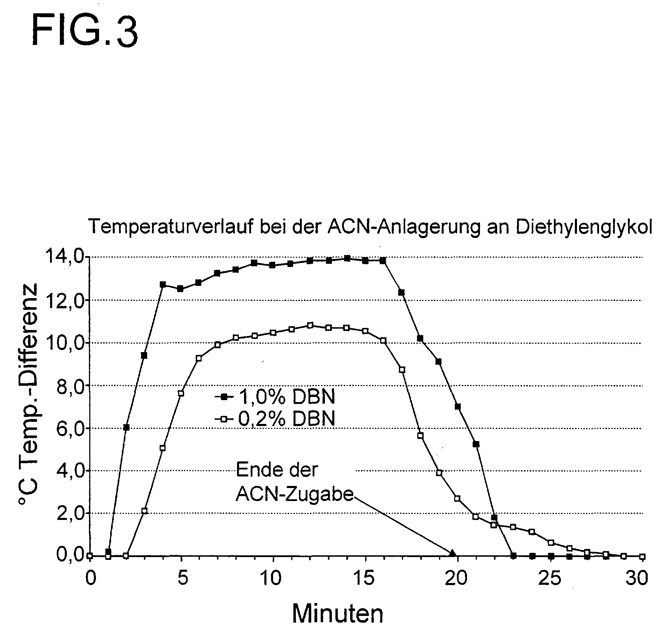

Diagramm 3 zeigt exemplarisch den Temperaturverlauf bei der Anlagerung mit 1 % und 0,2 % DBN, vergleichbare Kurven wurden auch mit den anderen Mengen erhalten.

### Beispiel 7: Methanol

48,5 kg Methanol (1515 mol) und 0,48 Gew.-% DBU wurden vorgelegt und bei 55 °C langsam (5 %/Min.) 74,3 kg Acrylnitril (1401 mol) hinzugefügt. Die Mischung wurde noch zwei Stunden bei 55 °C nachreagieren gelassen. Das 3-Methoxypropionitril wurde dann an einem Kobalt-Katalysator bei 110 °C kontinuierlich hydriert, die Austräge destilliert und analysiert. Eingesetzt wurden 250 ml Katalysator, die bei 20 MPa Wasserstoffdruck mit 75 ml • h⁻¹ Methoxypropionitril und 500 ml • h⁻¹ NH₃ beaufschlagt wurden. Die Belastung betrug 0,3 1_{Eduktl}1_{Kat}.⁻¹h⁻¹.

Die nach destillativer Aufarbeitung bestimmte Ausbeute an 3-Methoxypropylamin betrug über einen Zeitraum von 14 Tagen konstant 95,9 %.

### Beispiel 8: Methanol

48,5 kg Methanol (1515 mol) und 0,48 Gew.-% DBN wurden vorgelegt und bei 55 °C langsam (5 %/Min.) 74,3 kg Acrylnitril (1401 mol) hinzugefügt. Die Mischung wurde noch zwei Stunden bei 55 °C nachreagieren gelassen. Das 3-Methoxypropionitril wurde dann an einem Kobalt-Katalysator bei 110 °C kontinuierlich hydriert, die Austräge destilliert und analysiert. Eingesetzt wurden 250 ml Katalysator, die bei 20 MPa Wasserstoffdruck mit 75 ml • h⁻¹ Methoxypropionitril und 500 ml • h⁻¹ NH₃ beaufschlagt wurden. Die Belastung betrug 0,3 1_{Edukt}1_{Kat}.⁻¹h⁻¹.

Die nach destillativer Aufarbeitung bestimmte Ausbeute an 3-Methoxypropylamin betrug über einen Zeitraum von 14 Tagen konstant 96,9 %.

## Patentansprüche

1. Verfahren zur Herstellung von β-Alkoxy-nitrilen durch Umsetzung von α,β-ungesättigten Nitrilen mit ein-, zwei- oder dreiwertigen Alkoholen in Gegenwart basischer Katalysatoren bei Temperaturen von -20 bis +200 °C, **dadurch gekennzeichnet, daß** man als Katalysator ein Diazabicycloalken der Formel I 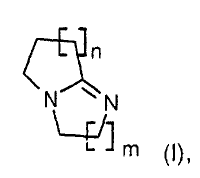 in der 1 bis 4 H-Atome unabhängig voneinander durch die Reste R¹ bis R⁴, wobei R¹, R², R³, R⁴ C₁₋₂₀-Alkyl, C₆₋₂₀-Aryl oder C₇₋₂₀-Arylalkyl bedeuten,
substituiert sein können sowie n und m ganze Zahlen von 1 bis 6 bedeuten, verwendet.

2. Verfahren nach Anspruch 1, **dadurch gekennzeichnet, daß** man als Katalysator ein Diazabicycloalken der Formel Ia 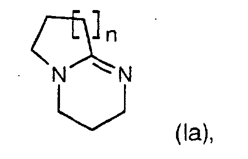 in der 1 bis 4 H-Atome unabhängig voneinander durch die Reste R¹ bis R⁴, wobei R¹, R², R³, R⁴ C₁₋₂₀-Alkyl, C₆₋₂₀-Aryl oder C₇₋₂₀-Arylalkyl bedeuten,
substituiert sein können sowie n eine ganze Zahl von 1 bis 3 bedeutet, verwendet.

3. Verfahren nach Anspruch 1, **dadurch gekennzeichnet, daß** man als Katalysator 1,5-Diazabicyclo[4.3.0]nonen-5 (DBN), 1,5-Diazabicyclo[4.4.0]decen-5 (DBD) oder 1,8-Diazabicyclo[5.4.0]undecen-7 (DBU) verwendet.

4. Verfahren nach den Ansprüchen 1 bis 3, **dadurch gekennzeichnet, daß** der Katalysator in einer Menge von 0,05 bis 5 Gew.-%, bezogen auf den Alkohol, eingesetzt wird.

5. Verfahren nach den Ansprüchen 1 bis 4, **dadurch gekennzeichnet, daß** man die Umsetzung bei Temperaturen von 25 bis 100 °C durchführt.

6. Verfahren zur Herstellung von γ-Alkoxy-aminen durch
a) Umsetzung von α,β-ungesättigten Nitrilen mit ein-, zweioder dreiwertigen Alkoholen in Gegenwart basischer Katalysatoren bei Temperaturen von -20 bis +200 °C zu β-Alkoxy-nitrilen und
b) anschließende Hydrierung der β-Alkoxynitrile in Gegenwart eines Hydrierkatalysators,
**dadurch gekennzeichnet, daß** im ersten Schritt als Katalysator ein Diazabicycloalken der Formel I gemäß den Ansprüchen 1 bis 3 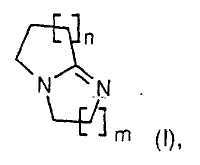 in der 1 bis 4 H-Atome unabhängig voneinander durch die Reste R¹ bis R⁴, wobei R¹, R², R³, R⁴ C₁₋₂₀-Alkyl, C₆₋₂₀-Aryl oder C₇₋₂₀-Arylalkyl bedeuten,
substituiert sein können sowie n und m ganze Zahlen von 1 bis 6 bedeuten, eingesetzt wird und die Hydrierung im zweiten Schritt in Gegenwart eines Hydrierkatalysators und des Katalysators der Formel I stattfindet.

## Claims

1. A process for preparing β-alkoxynitriles by reacting α,β-unsaturated nitriles with monohydric, dihydric or trihydric alcohols in the presence of basic catalysts at from -20 to +200°C, which comprises using a diazabicycloalkene catalyst of the formula I 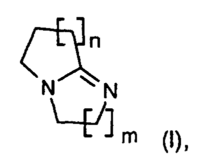 where from 1 to 4 hydrogen atoms may be independently replaced by the radicals R¹ to R⁴, in which case R¹, R², R³, R⁴ are each C₁₋₂₀-alkyl, C₆₋₂₀-aryl or C₇₋₂₀-arylalkyl, and
n and m are each an integer from 1 to 6.

2. A process as claimed in claim 1, wherein the catalyst used is a diazabicycloalkene of the formula Ia 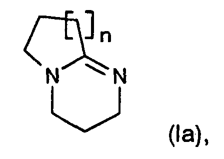 where from 1 to 4 hydrogen atoms may be independently replaced by the radicals R¹ to R⁴, in which case R¹, R², R³, R⁴ are each C₁₋₂₀-alkyl, C₆₋₂₀-aryl or C₇₋₂₀-arylalkyl, and
n is an integer from 1 to 3.

3. A process as claimed in claim 1, wherein the catalyst used is 1,5-diazabicyclo[4.3.0]non-5-ene (DBN), 1,5-diazabicyclo-[4.4.0]dec-5-ene (DBD) or 1,8-diazabicyclo[5.4.0]undec-7-ene (DBU).

4. A process as claimed in any of claims 1 to 3, wherein the catalyst is used in an amount of from 0.05 to 5% by weight, based on the alcohol.

5. A process as claimed in any of claims 1 to 4, wherein the reaction is carried out at from 25 to 100°C.

6. A process for preparing γ-alkoxyamines by
a) reaction of α,β-unsaturated nitriles with monohydric, dihydric or trihydric alcohols in the presence of basic catalysts at from -20 to +200°C to form β-alkoxynitriles, and
b) subsequent hydrogenation of the β-alkoxynitriles in the presence of a hydrogenation catalyst,
which comprises using in the first step a diazabicycloalkene catalyst of the formula I as set forth in any of claims 1 to 3 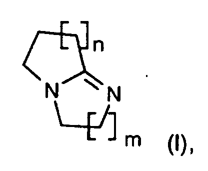 where from 1 to 4 hydrogen atoms may be independently replaced by the radicals R¹ to R⁴, in which case R¹, R², R³, R⁴ are each C₁₋₂₀-alkyl, C₆₋₂₀-aryl or C₇₋₂₀-arylalkyl, and
n and m are each an integer from 1 to 6, and effecting the hydrogenation in the second step in the presence of a hydrogenation catalyst and of the catalyst of the formula I.

## Revendications

1. Procédé pour la préparation de β-alcoxy-nitriles au moyen d'une réaction de nitriles à insaturation en position α,β avec des mono-, di- ou triols, en présence des catalyseurs basiques, à une température comprise entre -20°C et +200°C, **caractérisé en ce que** l'on met en oeuvre, en tant que catalyseur, un diazabicycloalcène de formule I 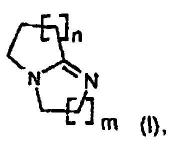 dans laquelle 1 à 4 atomes de H sont éventuellement substitués, indépendamment les uns des autres, par des restes R¹ à R⁴, où R¹, R², R³ et R⁴ représentent un groupe alkyle en C₁ à C₂₀, aryle en C₆ à C₂₀ ou arylalkyle en C₇ à C₂₀, et n et m représentent un nombre entier compris entre 1 et 6.

2. Procédé selon la revendication 1, **caractérisé en ce que** l'on met en oeuvre, en tant que catalyseur, un diazabicycloalcène de formule Ia 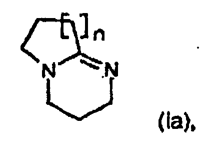 dans laquelle 1 à 4 atomes de H sont éventuellement substitués, indépendamment les uns des autres, par des restes R¹ à R⁴, où R¹, R², R³ et R⁴ représentent un groupe alkyle en C₁ à C₂₀, aryle en C₆ à C₂₀ ou arylalkyle en C₇ à C₂₀, et n représente un nombre entier compris entre 1 et 3.

3. Procédé selon la revendication 1, **caractérisé en ce que** l'on met en oeuvre, en tant que catalyseur,
le 1,5-diazabicyclo[4.3.0]nonène-5 (DBN),
le 1,5-diazabicyclo[4.4.0]décène-5 (DBD) ou
le 1,8-diazabicyclo[5.4.0]undécène-7 (DBU).

4. Procédé selon les revendications 1 à 3, **caractérisé en ce que** l'on met en oeuvre le catalyseur à raison de 0,05% à 5% en poids, par rapport à l'alcool.

5. Procédé selon les revendications 1 à 4, **caractérisé en ce que** l'on procède à la réaction à une température comprise entre 25°C et 100°C.

6. Procédé pour la préparation de γ-alcoxy-amines au moyen de
a) une réaction de nitriles à insaturation en position α,β avec des mono-, di- ou triols, en présence de catalyseurs basiques, à une température comprise entre -20°C et +200°C, pour obtenir des β-alcoxy-nitriles,
b) suivie d'une hydrogénation des β -alcoxy-nitriles en présence d'un catalyseur d'hydrogénation,
**caractérisé en ce que**, dans la première étape, on met en oeuvre, en tant que catalyseur, un diazabicycloalcène de formule I selon les revendications 1 à 3 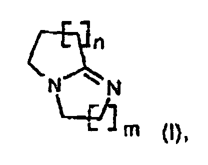 formule dans laquelle 1 à 4 atomes de H sont éventuellement substitués, indépendamment les uns des autres, par des restes R¹ à R⁴, où R¹, R², R³ et R⁴ représentent un groupe alkyle en C₁ à C₂₀, aryle en C₆ à C₂₀ ou arylalkyle en C₇ à C₂₀,
et n et m représentent un nombre entier compris entre 1 et 6, et **en ce que**, dans la deuxième étape, on réalise l'hydrogénation en présence d'un catalyseur d'hydrogénation et du catalyseur de formule I.
